# EUROPÄISCHE PATENTANMELDUNG

(11) **EP 3 255 138 A1**
(43) Veröffentlichungstag der Anmeldung: **13.12.2017**
(21) Anmeldenummer: 17174780.1
(22) Anmeldetag: 07.06.2017
(51) Int. Cl.: C12M 1/107, C12M 1/26, C12M 1/34

(54) **VORRICHTUNG ZUM ENTNEHMEN ZUMINDEST EINER BIOMASSE-PROBE AUS WENIGSTENS EINEM FERMENTER INSBESONDERE EINER BIOGASANLAGE**

(30) Priorität: 07.06.2016 DE 102016110482
(71) Anmelder: bwe Energiesysteme GmbH & Co. KG, 26169 Friesoythe (DE)
(72) Erfinder: Wilches Tamayo, Camilo Andrés, 26129 Oldenburg (DE)
(74) Vertreter: Jabbusch, Matthias

(57) **Zusammenfassung**

Bei einer Vorrichtung zum Entnehmen zumindest einer Biomasse-Probe aus wenigstens einem Fermenter insbesondere einer Biogasanlage, wobei an den Fermenter zumindest eine Biomasse-Leitung angeschlossen ist, der zumindest eine Förderpumpe für die Biomasse zugeordnet ist, ist vorgesehen, dass an die Biomasse-Leitung in einem Abschnitt auf der Druckseite der Förderpumpe zumindest zwei Abzweigleitungen angeschlossen sind, die in eine gemeinsame Abzweigleitung einmünden, wobei sich diese gemeinsame Abzweigleitung in zumindest zwei Teilleitungen aufzweigend ausgebildet ist und in der Abzweigungsleitung und in jeder Teilleitung ein Leitungsschließelement angeordnet ist.

Mit dieser Vorrichtung kann eine definierte Menge an Biomasse aus voneinander verschiedenen Regionen einzelner oder mehrerer Fermenter entnommen werden.

## Beschreibung

Die Erfindung betrifft eine Vorrichtung zum Entnehmen zumindest einer Biomasse-Probe aus wenigstens einem Fermenter insbesondere einer Biogasanlage, wobei an den Fermenter zumindest eine Biomasse-Leitung angeschlossen ist, der zumindest eine Förderpumpe für die Biomasse zugeordnet ist.

Biogasanlagen mit einem oder mehreren Fermentern sind inzwischen für die Erzeugung von elektrischer Energie weit verbreitet. Die Biogasanlagen bestehen aus Fermentern, ihnen werden pflanzliche und andere Stoffe zugeführt. In den Fermentern befindliche Biomasse ist regelmäßig von einem Fermenter in einen anderen Fermenter zu überführen, schließlich sind Reststoffe aus dem Fermenter abzuführen. Dazu dienen oben angegebene Biomasse-Leitungen, denen Förderpumpen zur Förderung der Biomasse zugeordnet sind.

Für die Überwachung der in den Fermentern ablaufenden Prozesse sind regelmäßig Biomasse-Proben aus den Fermentern zu entnehmen. Dies erfolgt im Stand der Technik durch das Öffnen eines am Fermenter befindlichen Hahnes, um dort Biomasse in ein Probengefäß entnehmen zu können. Diese Entnahme ist mit dem Nachteil behaftet, dass sie nur an einem Ort des Fermenters entnommen werden kann, nämlich dem Ort des Hahnes. Somit kann keine repräsentative Probe entnommen werden, die den gesamten Inhalt des Fermenters widergibt. Die Probe wird nach der Entnahme manuell für die Analyse vorbereitet. Dazu wird diese mit einem gewöhnlichen Sieb von groben Partikeln befreit.

Der Erfindung liegt die Aufgabe zugrunde, eine Vorrichtung der eingangs genannten Gattung aufzuzeigen, mit der eine definierte Menge an Biomasse aus voneinander verschiedenen Regionen eines oder mehrerer Fermenter entnommen werden kann.

Diese Aufgabe ist erfindungsgemäß dadurch gelöst, dass an die Biomasse-Leitung in einem Abschnitt auf der Druckseite der Förderpumpe zumindest zwei Abzweigleitungen angeschlossen sind, die in eine gemeinsame Abzweigleitung einmünden, wobei sich diese gemeinsame Abzweigleitung in zumindest zwei Teilleitungen aufzweigend ausgebildet ist und in der Abzweigungsleitung und in jeder Teilleitung ein Leitungsschließelement angeordnet ist.

Nach der Erfindung ist vorgesehen, Biomasse für eine Probe nicht direkt aus dem Fermenter zu entnehmen, sondern aus einer Biomasse-Leitung. Durch die Biomasse-Leitung wird Biomasse aus allen Bereichen eines oder mehrerer Fermenter gefördert. Die Entnahme soll auf der Druckseite einer in die Biomasse-Leitung eingesetzten Pumpe erfolgen, obwohl hier ein hoher Druck herrscht.

Zur Entnahme einer oder mehrerer Biomasse-Proben ist die Abzweigleitung vorgesehen. Diese zweigt sich in zumindest zwei Teilleitungen auf, beide Teilleitungen sind mit Leitungsschließelementen ausgerüstet.

Auch die Abzweigleitung ist mit einem Leitungsschließelement ausgerüstet, wird dies geöffnet, tritt in die Abzweigleitung Biomasse ein, die bis in die beiden Teilleitungen fließt. Dort wird das in den Teilleitungen vorhandene Luftpolster komprimiert. Der im Luftpolster herrschende Druck entspricht schließlich dem Förderdruck der Pumpe, so dass eine weitere Förderung der Biomasse in die Abzweigleitung hinein nicht stattfindet. Das in der Abzweigleitung angeordnete Leitungsschließelement kann jetzt geschlossen werden, so dass die in der Abzweigleitung hinter dem Leitungsschließelement und in den Teilleitungen angeordnete Biomasse als Probe zur Verfügung steht und abgeführt werden kann.

Trotz der Druckverhältnisse in der Biomasse-Leitung ist es mit der erfindungsgemäßen Vorrichtung möglich, eine Biomasse-Probe zu erhalten. Es können auch nacheinander mehrere Biomasse-Proben abgeführt werden, da nach Entleerung und Einstellen von Normaldruckverhältnissen in den Teilleitungen hinter dem Leitungsschließelement in der Abzweigleitung die Leitungsschließelemente in den Teilleitungen wieder geschlossen werden können und nachfolgend durch Öffnen des Leitungsschließelementes in der Abzweigleitung wieder Biomasse in die Teilleitungen einfließen kann.

Nach einer ersten Weiterbildung der Erfindung ist vorgesehen, dass der Biomasse-Leitung ein Drucksensor zugeordnet ist. Mit diesem kann der Druck in der Biomasse-Leitung überwacht werden, dieser Druck stellt sich auch in der Abzweigleitung und in den Teilleitungen ein, wenn während ein in den Teilleitungen angeordnetes Luftvolumen komprimiert wurde.

Bei durch den Drucksensor bekannten Druckverhältnissen in der Biomasse-Leitung und in dem Luftpolster sind mit der erfindungsgemäßen Vorrichtung Biomasse-Probenvolumina exakt bestimmbar. Das Volumen der Abzweigleitung hinter dem Leitungsschließelement und der zwei Teilleitungen von dem jeweiligen Leitungsschließelementen ist bekannt und kann bei bekannten Druckverhältnissen in entsprechende Volumenbestimmungen für Biomasse-Proben eingerechnet werden.

Nach einer nächsten Weiterbildung der Erfindung ist vorgesehen, dass zumindest eine Teilleitung in einen Probensammelbehälter mündet und dass in dieser Teilleitung und in der anderen Teilleitung atmosphärische Druckverhältnisse herstellbar sind. Atmosphärische Druckverhältnisse werden vor dem Entnehmen einer Biomasse-Probe hergestellt. Zu diesem Zeitpunkt ist eine ggfs. vorher gewonnene Probe in den Probensammelbehälter überführt worden.

Dem Probensammelbehälter ist vorzugsweise ein Druckgasanschluss, beispielsweise ein Druckluftanschluss zugeordnet. Ein Druckgas kann in den Probensammelbehälter eingeführt werden, um in diesem aufgenommene mehrere Biomasse-Proben miteinander zu verwirbeln und damit zu vermischen. Somit kann insgesamt eine Biomasse-Probe erzeugt werden, die aus verschiedenen Einzelproben eines oder mehrerer Fermenter zusammengesetzt ist.

Zur weiteren konstruktiven Ausbildung der Erfindung ist vorgesehen, dass der Probensammelbehälter in einem Leitungsstrang mit zumindest einer dem Probensammelbehälter nachfolgenden Filtereinrichtung angeordnet ist. Mit der Filtereinrichtung kann die Biomasse-Probe gefiltert werden, dabei können mehrere Filtereinrichtungen mit im Verlauf des Leitungsstranges kleiner werdenden Korngrößen vorgesehen sein. Möglich ist eine kleine Korngröße von beispielsweise 0,1 mm.

Die gewonnene Biomasse-Probe kann dann auf übliche Weise bearbeitet und ausgewertet werden, beispielsweise mit einer Titriereinrichtung.

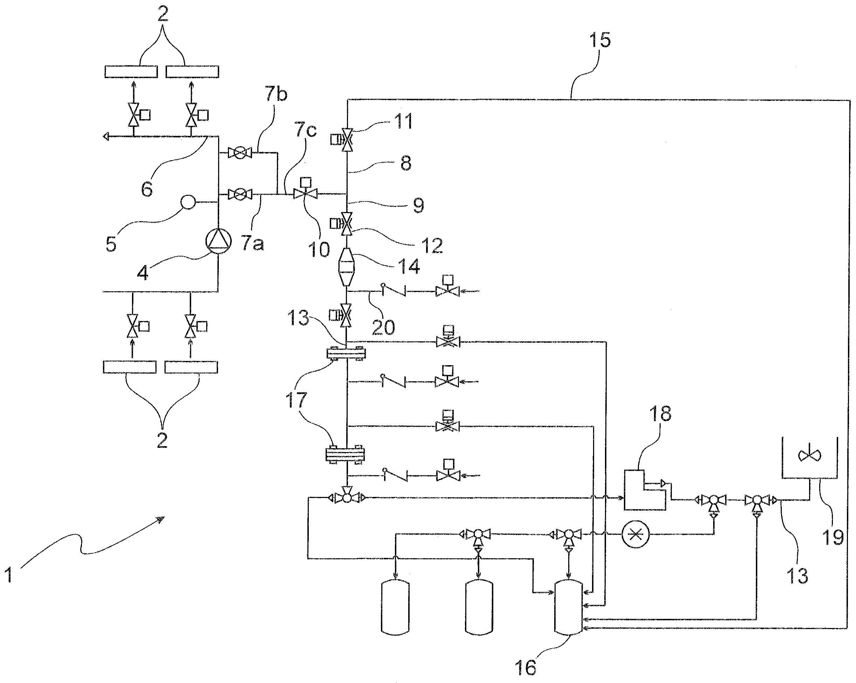

Ein Ausführungsbeispiel der Erfindung, aus dem sich weitere erfinderische Merkmale ergeben, ist in der Zeichnung dargestellt. Die einzige Figur der Zeichnung zeigt eine schematische Ansicht einer erfindungsgemäßen Vorrichtung zum Entnehmen zumindest einer Biomasse-Probe aus wenigstens einem Fermenter insbesondere einer Biogasanlage.

Die in der Figur gezeigte Biogasanlage 1 hat zwei Fermenter 2. Die Fermenter 2 sind jeweils an der Saugseite und an der Druckseite an eine Biomasse-Leitung 6 angeschlossen. In diese Biomasse-Leitung 6 ist eine Förderpumpe 4 eingesetzt, weiterhin ist der Biomasse-Leitung 6 eine Druckmesseinrichtung 5 zugeordnet. Mit der Förderpumpe 4 kann Biomasse zwischen den Fermentern 2 gefördert werden.

Auf der Druckseite der Förderpumpe 4 sind an die Biomasse-Leitung 6 zwei Abzweigleitungen 7a, 7b angeschlossen. Diese münden in eine Abzweigleitung 7c, die sich in zwei Teilleitungen 8 und 9 aufzweigt. In der Abzweigleitung 7c und in den Teilleitungen 8, 9 sind erfindungsgemäß Leitungsschließelemente 10, 11, 12 angeordnet.

Zum Abzweigen einer Biomasse-Probe aus der Biomasse-Leitung 6 wird das Leitungsschließelement 10 geöffnet, während die Leitungsschließelemente 11 und 12 geschlossen sind. Zwischen den Leitungsschließelementen 10, 11, 12 wurde zuvor atmosphärischer Druck hergestellt. Beim Betrieb der Förderpumpe 4 wird Biomasse vom Fermenter 2 über die Leitung 6 gefördert. Durch den Einbau der Abzweigleitungen 7a und 7b kann nun parallel Biomasse transportiert werden, durch die höhere Fließgeschwindigkeit in Leitung 6 wird die Biomasse vermischt und über die Leitung 7c bis zum Leitungsschließelement 10 gefördert.

Nach Öffnen des Leitungsschließelementes 10 fließt Biomasse über die Abzweigleitung 7c in die Teilleitungen 8 und 9 bis zu den geschlossenen Leitungsschließelementen 11, 12. Beim Leitungsschließelement 11 bildet sich ein komprimiertes Luftvolumen aus. Jetzt kann das Leitungsschließelement 10 in der Abzweigleitung 7c geschlossen werden, so dass die zwischen den Leitungsschließelementen 10, 11, 12 befindliche Probe von der Biomasse-Leitung 6 abgetrennt ist. Nach Öffnen des Leitungsschließelementes 12 kann diese Probe in einen in einem Leitungsstrang 13 befindlichen Probensammelbehälter 14 abfließen. Nach Schließen des Leitungsschließelementes 12 und Öffnen des Leitungs-schließelementes 11 können im Probensammelbereich zwischen den Leitungsschließelementen 10, 11, 12 wieder atmosphärische Druckverhältnisse hergestellt werden.

Dem Probensammelbehälter 14 ist ein Druckgasanschluss 20 zugeordnet. Mit diesem kann ein Gas unter Druck, beispielsweise Druckluft, in den Probensammelbehälter 14 zum Vermischen der dort angeordneten Biomasse-Proben eingeführt werden.

Im Leitungsstrang 13 sind nachfolgend Filtereinrichtungen 17 angeordnet. Schließlich ist die Probe über verschiedene Ventile und eine Dosierpumpe 18 einer Titriereinrichtung 19 zuführbar.

## Patentansprüche

1. Vorrichtung zum Entnehmen zumindest einer Biomasse-Probe aus wenigstens einem Fermenter insbesondere einer Biogasanlage, wobei an den Fermenter zumindest eine Biomasse-Leitung angeschlossen ist, der zumindest eine Förderpumpe für die Biomasse zugeordnet ist,
**dadurch gekennzeichnet,**
**dass** an die Biomasse-Leitung (3) in einem Abschnitt auf der Druckseite der Förderpumpe (4) zumindest zwei Abzweigleitungen (7a, 7b) angeschlossen sind, die in eine gemeinsame Abzweigleitung (7c) einmünden, wobei sich diese gemeinsame Abzweigleitung (7c) in zumindest zwei Teilleitungen (8, 9) aufzweigend ausgebildet ist und in der Abzweigungsleitung (7c) und in jeder Teilleitung (8, 9) ein Leitungsschließelement (10, 11, 12) angeordnet ist.

2. Vorrichtung nach Anspruch 1, **dadurch gekennzeichnet, dass** der Biomasse-Leitung (3) eine Druckmesseinrichtung zugeordnet ist.

3. Vorrichtung nach Anspruch 1 oder 2, **dadurch gekennzeichnet, dass** zumindest eine Teilleitung (9) in einen Probensammelbehälter (14) mündet und dass in dieser und in der anderen Teilleitung (8) atmosphärische Druckverhältnisse herstellbar sind.

4. Vorrichtung nach Anspruch 3, **dadurch gekennzeichnet, dass** dem Probensammelbehälter (14) ein Druckgasanschluss (20) zugeordnet ist.

5. Vorrichtung nach Anspruch 3 oder 4, **dadurch gekennzeichnet, dass** der Probensammelbehälter (14) in einem Leitungsstrang (13) mit zumindest einer dem Probensammelbehälter (14) nachfolgenden Filtereinrichtung (17) angeordnet ist.

6. Vorrichtung nach Anspruch 5, **dadurch gekennzeichnet, dass** mehrere Filtereinrichtungen (17) mit im Verlauf des Leitungsstrangs (13) kleiner werdenden Korngrößen vorgesehen sind.

7. Vorrichtung nach Anspruch 6, **dadurch gekennzeichnet, dass** eine Filtereinrichtung (17) mit einer Korngröße von 0,1 mm vorgesehen ist.

8. Vorrichtung nach einem der Ansprüche 4 bis 7, **dadurch gekennzeichnet, dass** der Leitungsstrang (13) zu einer Titriereinrichtung (19) oder anderer Messanalytik geführt ist.
